# EUROPEAN PATENT SPECIFICATION

(11) **EP 1 715 913 B1**
(45) Date of publication and mention of the grant of the patent: **21.12.2011**
(21) Application number: 05713433.0
(22) Date of filing: 08.02.2005
(51) Int. Cl.: A61M 25/10

(54) **APPARATUS FOR CREATING WORKING CHANNEL TROUGH TISSUE**
GERÄT ZUR ERZEUGUNG EINES ARBEITSKANALS DURCH GEWEBE
APPAREIL DE CREATION D'UN CANAL OPERATEUR A TRAVERS UN TISSU

(30) Priority: 18.02.2004 US 782359
(43) Date of publication of application: 02.11.2006
(73) Proprietor: Stryker Corporation, Kalamazoo, MI 49002 (US); Stryker NV Operations Limited, Dublin 2 (IE)
(72) Inventor: CHOPRA, Gopal, Seattle, WA 98122 (US)
(74) Representative: Hauck Patent- und Rechtsanwälte
(86) International application number: PCT/US2005/004494
(87) International publication number: WO 2005/079905

(56) References cited:
- WO-A-96/38109
- US-A- 5 378 237
- US-A- 5 487 739

## Description

### FIELD OF THE INVENTION

The invention relates to medical devices, and in particular, medical devices for accessing tissue and creating access pathways in tissue to a target site in the brain.

### BACKGROUND OF THE INVENTION

Many medical procedures require access to deep brain tissue. For instance, it is known to surgically treat neurodegenerative diseases, such as Tumors, Alzheimer's Disease, Parkinson's Disease, Tremor, and Epilepsy, and ischemia of the brain, such as stroke, with procedures such as ablative surgery or restorative surgery. Ablative and resection surgeries for tumor are common in the practice of neurosurgery for debulking a lesion for further adjunctive therapy or to alleviate pressure build up resulting from a mass in the skull. The tumor is removed using ablative therapies such as bipolar and laser, as well as resected with suction, excision or treatment with cavitronic ultrasound devices. The tumors are usually deep below the surface of the brain and normal brain tissue is often displaced to reach the target lesion with the assistance of significant localization technologies.

Some medical procedures for the treatment of stroke victims also require access to deep brain tissue. Strokes often leave clots in the brain called intracerebral hematomas. These hematomas can be removed by highly invasive surgery. They can also be removed by drainage after minimally invasive surgery. Once stabilized, the offending source or cause of the hematoma is addressed.

Although current surgical techniques have proven successful in the treatment of brain disorders, such techniques are still quite invasive, requiring access to deep brain tissue. Often, more superficial brain tissues are sacrificed while accessing deeper tissues. Typical devices used to gain access to deep tissue targets include large obturators with sharp piercing tips that cut superficial brain tissues overlying the deeper tissue targets. Cutting superficial brain tissues can cause significant damage. The trajectory is often calculated and guided with image based navigation systems that fuse historical imaging data with surface markers on the patient, increasing accuracy of the pathway to the target, guiding the surgeon through treacherous territory.

US patent No. 5,487,739 to Aebischer et al. discloses an implantable therapy system including a dilator (item 104 in Aebischer et al.) used to increase the size of an insertion site in a body in order to gain access to the treatment site by spreading apart the tissue".

### SUMMARY OF THE INVENTION

In one embodiment of the invention, a medical device comprises an elongated member, which may be, e. g. , introduced through tissue. Preferably, the elongated member has a blunt tip in order to minimize tissue trauma. The device further comprises a first radially expandable body (e. g. , an inflatable balloon) surrounding the distal end of the elongated member, and a stent surrounding the first body. In one embodiment, the elongated member and first body can be removed from the device, thereby creating a working channel within the stent to allow medical instruments or medicaments to be placed through the stent. In this case, the stent is preferably plastically deformable to maintain the working channel created by the removal of the elongated member.

The device further comprises a second radially expandable body (e. g, an expandable annular balloon) surrounding the stent. The second expandable body can, e. g. , provide a means for radially displacing tissue along the path. The device may optionally comprise a haemostatic coating surrounding the second body to, e. g., prevent or minimize bleeding of tissue.

### BRIEF DESCRIPTION OF THE DRAWINGS

The drawings illustrate the design and utility of an exemplary embodiment of the invention, and in which:
**Fig. 1** is a side view of an invasive medical device constructed in accordance with one embodiment of the invention;
**Fig. 2** is a cross sectional view, along the axis, of the distal end of the device of **Fig. 1****;**
**Fig. 3** is a cross sectional view, perpendicular to the axis, of the distal end of the device of **Fig. 1****;**
**Figs. 4A-4G** are cross sectional views, along the axis of the medical device, illustrating a method of treating a tissue using the medical device of Fig. 1; and
**Figs. 5A-5G** are cross sectional views, perpendicular to the axis of the medical device, illustrating a method of treating a tissue using the medical device of **Fig. 1****.**

### DETAILED DESCRIPTION OF THE ILLUSTRATED EMBODIMENTS

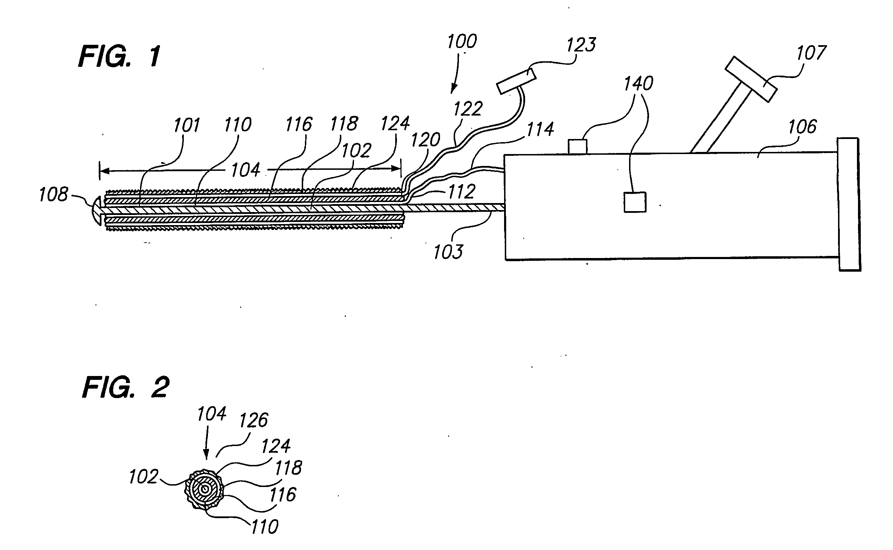

Referring to **Fig. 1****,** a medical device 100 constructed in accordance with one embodiment of the invention is shown. In its simplest form, the device 100 comprises an elongated guide member 102 having a distal end 101 and a proximal end 103, a tissue channel forming assembly 104 mounted to the distal end 101 of the guide member 102, and a handle 106 mounted to the proximal end 103 of the guide member 102. The handle 106 can be ergonomically designed to allow a physician to more easily manipulate the device 100, and optionally features an injection port 107. Two emitters 140, that are separated from each other, are mounted onto the medical device 100 to allow guidance by a navigation system (not shown).

As will be described in greater detail below, the channel forming assembly 104 is configured to open a working channel in tissue through which surgical or diagnostic medical instruments or therapeutic agents can be introduced to reach a remote target tissue site. The tissue channel forming assembly 104 comprises two inflation tubes 114 and 122 through which an inflation medium is conveyed in order to actuate the channel forming assembly 104 in a manner described below. The first inflation tube 114 is connected to an inflation port 107 located on the handle 106, and the second inflation tube 122 is connected to a free inflation port 123. Alternatively, the first inflation tube 114 can be connected to a free inflation port (not shown), in which case, the inflation port 107 on the handle 106 will not be needed.

The guide member 102 can be composed of any suitable axially rigid or semirigid material, such as stainless steel, nitinol, etc. that allows the guide member 102 to be introduced through solid tissue. In this embodiment, the guide member 102 has a blunt tip 108 to minimize tissue damage during insertion. The length of the channel forming assembly 104, is preferably sized to approximate the length of the working channel that is to be formed through the tissue to reach the remote tissue target site. For example, exemplary lengths within the range of four to eight inches are typical. If the length of the channel forming assembly 104 is significantly greater than the length of the working channel, the difference in resistance to expansion may cause uneven expansion along the length of the channel forming assembly 104. The diameter of the guide member 102 is preferably equal to the minimum diameter necessary to maintain axial rigidity of the guide member 102. For example, exemplary diameters within the range of ten thousandths to forty thousandths of an inch are typical.

Referring to **Figs. 1** and **2****,** the channel forming assembly 104 will now be described in further detail. The channel forming assembly 104 is shown in **Figs. 1** and 2 as being in a low-profile fully collapsed state to aid in delivering the medical device 100 through the tissue. As will be described later, the channel forming assembly 104 can be placed into various stages of expanded states in order to create a working channel through tissue. To this end, the channel forming assembly 104 generally comprises an inner radially expandable body 110 surrounding the distal end 101 of the guide member 102, a stent 116 surrounding the inner body 110, and an outer radially expandable body 118 surrounding the stent 116.

In the illustrated embodiment, the inner body 110 takes the form of a balloon. The inner body 110 is preferably composed of a non-compliant or semi-compliant material, such as those typically used for angioplastly balloons. The inner body 110 may be formed of a wide variety of suitable compliant or non-compliant materials known in the art. However, for purposes of the invention, elastomeric polymers are preferred. Examples of suitable elastomers include silicone, latex, and thermoplastic polyolefin rubbers. Alternatively, the inner body 110 may be formed of a thermoplastic polyisoprene rubber such as hydrogenated polyisoprene. Thermoplastic polyisoprene rubber has a number of advantages in terms of both performance and manufacture over conventional elastomeric materials.

For example, silicone balloons tend to yield larger profiles due to manufacturing limitations associated with wall thickness. In addition, silicone balloons are expensive to manufacture and assemble, because they require specialized manufacturing equipment and are not easily bonded to conventional shaft materials. Similarly, latex balloons are difficult to bond to conventional shaft materials. Latex balloons are considered toxic and excessively compliant. Balloons formed of thermoplastic polyolefin rubbers typically have a larger profile due to manufacturing limitations associated with wall thickness. Specifically, thermoplastic polyolefin rubbers usually contain a dispersion of Ethylene Propylene Diene Monomer (EPDM) rubber, which limits how thin the balloon tubing may be extruded.

By contrast, balloons formed of thermoplastic polyisoprene rubber, such as hydrogenated polyisoprene have superior performance and manufacturing attributes. For example, hydrogenated polyisoprene, which is commercially available under the trade name CHRONOPRENE from CT Biomaterials, may be processed with standard polyolefin processing equipment to obtain balloon tubing having a wall thickness of approximately 0.003 inches to 0.010 inches and a corresponding inside diameter of approximately 0.016 inches to 0.028 inches. Such balloon tubing has been demonstrated to produce balloons having a nominal outside diameter when inflated of approximately 3.0 mm to 5.5 mm. The wall thickness of the balloon is on the order of 0.001 inches, which allows the balloon to have a very low deflated profile, which in turn allows for insertion of into tissue with minimal damage.

Balloons made from thermoplastic polyisoprene rubber inflate uniformly and typically form a cylindrical shape when inflated. The rupture pressure has been shown to be approximately one atmosphere, which is desirable for creating working channels in tissue. The thermoplastic polyisoprene rubber has also demonstrated superior manufacturing capabilities. Hydrogenated polyisoprene is readily bondable to conventional shaft materials and may be extruded using conventional extrusion equipment.

The inner body 110 can be chemically bonded to the elongated member 102 with an adhesive or the two elements can be heat bonded together. The inner body 110 defines a first port 112 connected to the first inflation tube 114, which in turn may be connected to a fluid source, such as a syringe for inflating and deflating the inner body 110. Fluid introduced into the first inflation tube 114 will travel through the first port 112 and into the inner body 110, thereby placing the inner body 110 into its expanded geometry. Fluid removed from the first inflation tube 114 will, in turn, remove the fluid from the inner body 110, thereby placing the inner body 110 into its collapsed geometry.

Preferably, the stent 116 has a complex geometry allowing it to be packed into a low profile collapsed state and stiff and stable enough radially, in an expanded state, to maintain patency of the working channel. The stent 116 may consist of any biocompatable material possessing the structural and mechanical attributes necessary for supporting the working channel. Thus, both metallic and polymeric materials are suitable. Examples of biocompatable metallic materials include stainless steel, tantalum, nitinol, and gold. Exemplary polymeric materials may be selected from the list immediately below, which is not exhaustive:
poly(L-lactide) (PLLA), poly(D,L-lactide) (PLA), polyglycolide (PGA), poly(L-lactide-co-D,L-lactide) (PLLA/PLA), poly(L-lactide-co-glycolide) (PLLA/PGA), poly(D, L-lactide-co-glycolide) (PLA/PGA), poly(glycolide-co-trimethylene carbonate) (PGA/PTMC), polyethylene oxide (PEO), polydioxanone (PDS), polycaprolactone (PCL), polyhydroxylbutyrate (PHBT), poly(phosphazene), polyD,L-lactide-co-caprolactone) (PLA/PCL), poly(glycolide-co-caprolactone) (PGA/PCL), polyanhydrides (PAN), poly(ortho esters), poly(phoshate ester), poly(amino acid), poly(hydroxy butyrate), polyacrylate, polyacrylamid, poly(hydroxyethyl methacrylate), elastin polypeptide co-polymer, polyurethane, polysiloxane and their copolymers.

The skeletal framework of the stent 116 may be formed through various methods as well. The framework may be welded, molded, or consist of filaments or fibers that are wound or braided together in order to form a continuous structure. The stent 116 is not bonded to the inner body 110, so that they may slide relative to each other along the longitudinal axis of the medical device 100. Once the stent 116 is expanded, it has sufficient strength to resist the inward pressure exerted by the tissue.

In this embodiment, the outer expandable body 118 takes the form of an annular balloon. The outer body 118 can be made of the same material as the inner body 110. The outer body 118 can be chemically bonded to the stent 116 with an adhesive or the two elements can be heat bonded together.

The outer body 118 defines a second port 120 connected to the second inflation tube 122, which in turn may be connected to a fluid source such as a syringe for inflating and deflating the outer body. Fluid introduced into the second inflation tube 122 will travel through the second port 120 and into the outer body 118, thereby placing the outer body 118 into its expanded geometry. Fluid removed from the second inflation tube 122 will, in turn, remove the fluid from the outer body 118, thereby placing the outer body 118 into its collapsed geometry. Because the first inflation tube 114 and the second inflation tube 122 are not connected to each other, the inner body 110 and outer body 118 can be expanded independently.

In the illustrated embodiment, the outer body 118 is surrounded by a haemostatic coating 124, which reduces bleeding when the device 100 is introduced through the tissue. The coating 124 can be a polymer that mechanically reduces bleeding. Alternatively, the coating 124 can contain a medicament that reduces bleeding, such as a vasoconstrictor or a coagulant. In alternative embodiments, the medical device 100 does not comprise a outer body 118, in which case, the haemostatic coating 124 may surround the stent 116.

As can be appreciated from **Fig. 2**, the haemostatic coating 124, outer radially expandable body 118, stent 116, inner radially expandable body 110, and elongated member 102 completely surround each other. In alternative embodiments, the coating 124 does not completely surround the outer body 118, but sufficiently surrounds the outer body 118 to reduce bleeding from the tissue through which the medical device 100 is inserted. Similarly, in alternative embodiments, the outer body 118 does not completely surround the stent 116, but sufficiently surrounds the stent 116 to create a space between the tissue and the stent 116. In yet other alternative embodiments, the stent 116 does not completely surround the inner body 110, but sufficiently surrounds the inner body 110 to resist inward pressure exerted by the tissue. In other alternative embodiments, the inner body 110 does not completely surround the elongated member 102, but sufficiently surrounds the elongated member 102 to exert pressure to expand the stent 116.

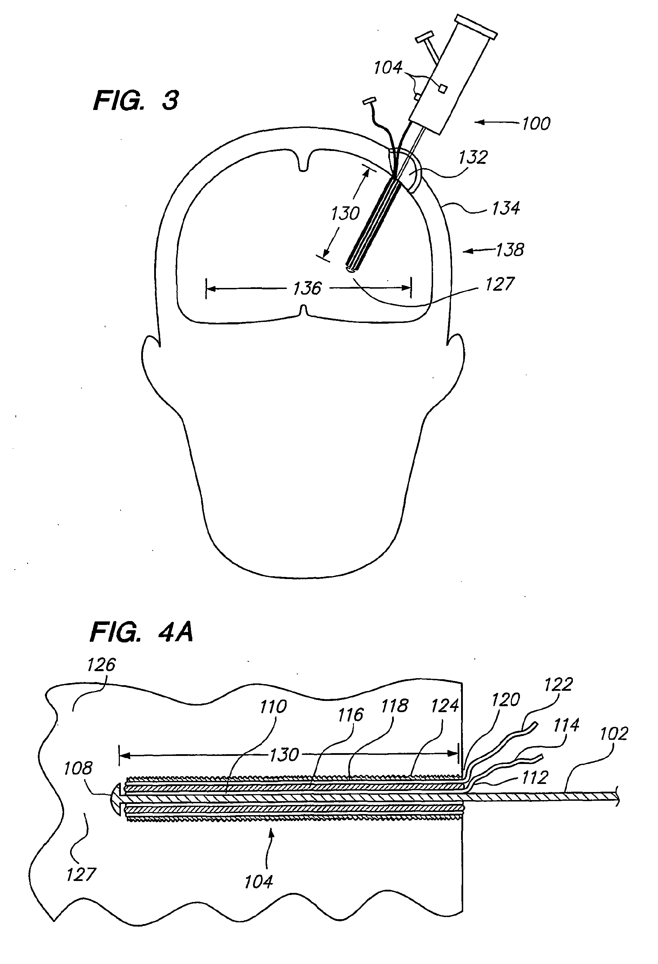

In order to better understand the structure of the medical device 100, its use to treat a remote tissue site 127, and in particular, a target site within the deep brain region 136 of a patient 138, will now be described with reference to **Figs. 3****, 4A-4G** and **5A-5G**.

A burr hole 132 is created within the cranium 134 of the patient 138 using standard techniques known in the art (**Fig. 3**). Then, the guide member 102, with the channel forming assembly 104 in its low profile and fully collapsed state, is introduced through the burr hole 132 and inserted through tissue 126 to create a path 130 to the remote tissue target site 127 (**Figs. 3, 4A** and **5A**). Two emitters 140, mounted on the device allows medical device 100 to be guided by a navigation system (not shown). To minimize tissue trauma, the guide member 102 is preferably introduced between naturally occurring tissue planes (not shown), which in the case of brain tissue, take the form of spaces between axons, which can be enlarged with minimal damage to the axons or neurons of which they are a part. As the channel forming assembly 104 is inserted into the tissue 126, the haemostatic coating 124 both minimizes bleeding from the tissue 126 and lubricates the path 130 to reduce frictional shearing forces during insertion.

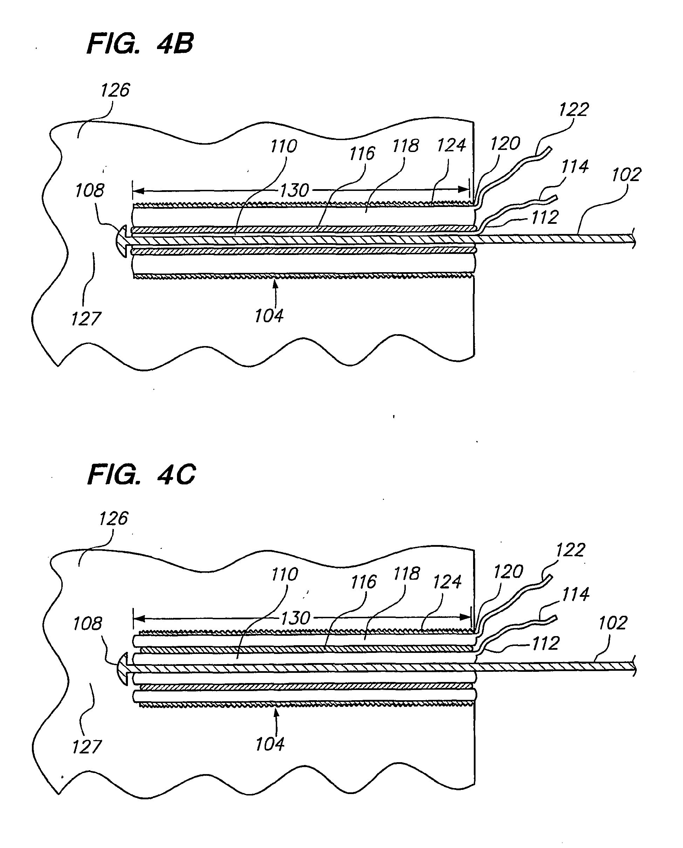

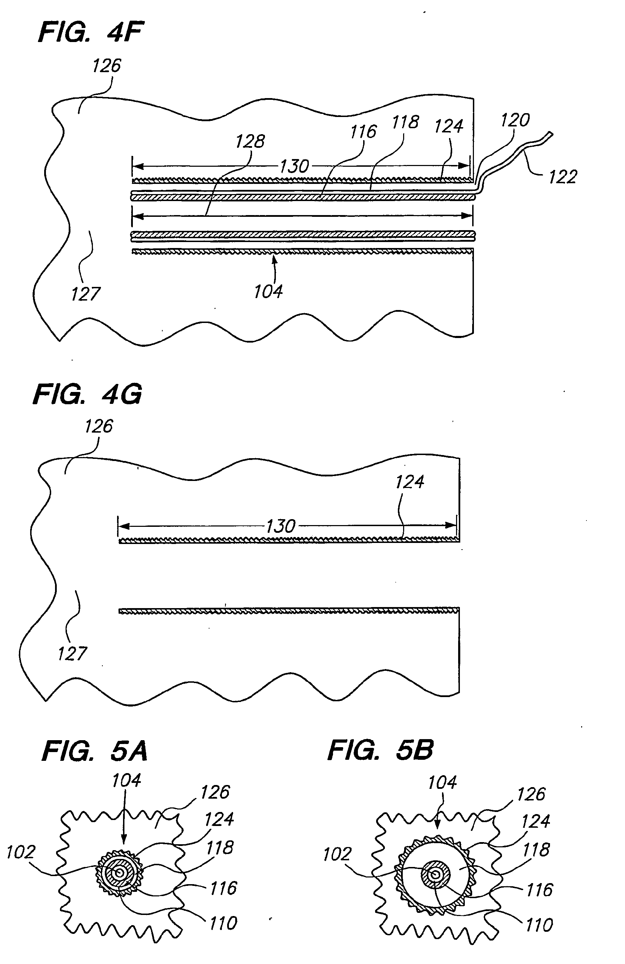

Once the channel forming assembly 104 is properly placed within the tissue 126, the outer body 118 is expanded to radially displace the brain tissue along the path 130 by conveying an inflation medium through the second inflation tube 122 and into the outer body 118 (**Fig. 4B** and **5B**). The outer body 118 may be incrementally expanded to prevent overly expanding the channel forming assembly 104 and damaging the tissue 126, e.g., by adding predetermined volumes of fluid. The outer body 118 may optionally' be expanded by filling it with a coolant, thereby reducing the temperature of the tissue 126 and further minimizing blood loss from the tissue 126.

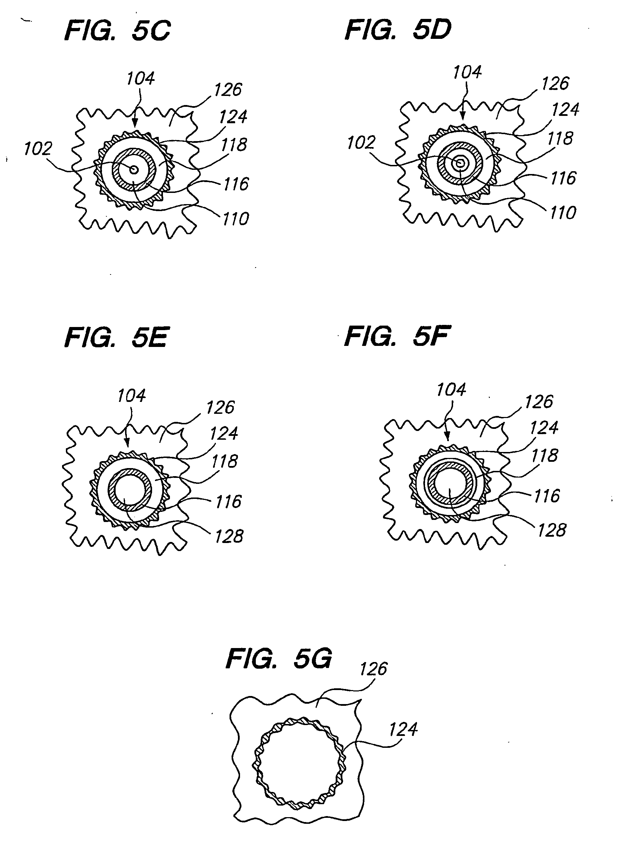

After the outer body 118 has been expanded, the inner body 110 is expanded by conveying an inflation medium through the first inflation tube 114 located in the handle 106 (shown in **Fig. 1**) and into the inner body 110 (**Fig. 4C** and **5C**), which in turn, expands the stent 116, thereby reinforcing the outer body 118 against the displaced tissue 126. The inner body 110 may be incrementally expanded to prevent overly expanding the channel forming assembly 104 and damaging the tissue 126, e.g., by adding predetermined volumes of the inflation medium (air or biocompatable fluid). The outer body 118 may be allowed to partially collapse by releasing fluid through the second port 120 into the second inflation tube 122, while the inner body 110 is expanded to reduce the pressure needed to expand the inner body 110 and the stent 116.

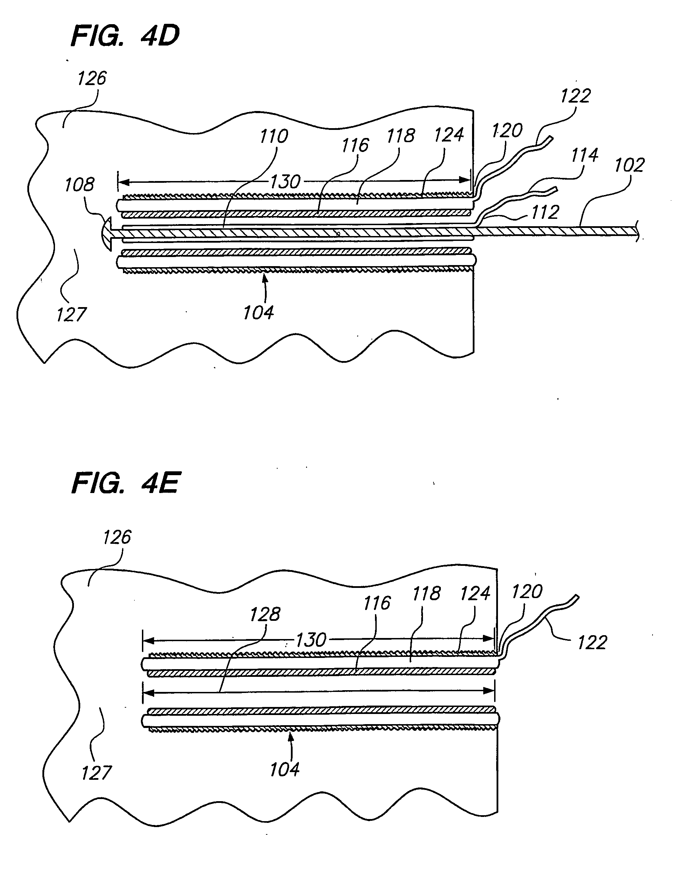

After the stent 116 is expanded to reinforce the radially displaced tissue, the inner body 110 is collapsed (**Figs. 4D** and **5D**) by releasing fluid through the first inflation tube 114 and out of the inflation port 107 on the handle 106 (both shown in Fig. 1). Because the stent 116 expands plastically, it maintains the patency of the working channel 128 despite the collapsing of the inner body 110 Once the inner body 110 is collapsed, the inner body 110 and the elongated member 102 are removed to form a working channel 128 through the stent 116. (**Figs. 4E** and **5E**) A medical element (not shown) is then introduced into the working channel 128 to perform a medical procedure on the tissue target site 127. Medical procedures that may be performed through the working channel 128 include ablative surgeries, restorative surgeries, and chemotherapy. Medical elements that may be introduced through the working channel 128 include ablation probes, needles, scalpels, and medicaments, such as chemotherapy agents. Diagnostic procedures can also be performed through the working channel 128.

After the procedure is concluded, the outer body 118 is collapsed by releasing fluid through the second inflation tube 122 and out of the inflation port 123 (shown in **Fig. 1****).** Once the outer body 118 is collapsed, it is removed from the working channel 128 **(****Figs. 4F** and **5F****).** Collapsing the outer body 118 reduces the profile of the tissue channel forming assembly 104 and allows it to be removed from the tissue 126 with minimal frictional shearing forces on the tissue 126. During removal of the tissue channel forming assembly 104, the haemostatic coating 124 acts as a lubricant to further minimize frictional shearing forces on the tissue 126. The haemostatic coating 124 is retained in the tissue 126 (see **Figs. 4G** and **5G****)** to minimize bleeding from the tissue 126.

## Claims

1. A medical device (100), comprising:
• an elongated member (102) having a proximal end (103) and a distal end (101);
• a first radially expandable body (110) surrounding the distal end (101) of the member (102);
• a stent (116) surrounding the first body (110), wherein radial expansion of the first body (110) radially expands the stent (116); and
• a second radially expandable body (118) surrounding the stent (116), wherein the first and second bodies (110, 118) are independently expandable.

2. The device of claim 1, wherein the distal end (101) of the member (102) is blunted.

3. The device of claims 1 or 2, wherein the first and second bodies (110, 118) are balloons.

4. The device of claims 1 or 2, wherein the first and second bodies (110, 118) expand elastically.

5. The device of any of claims 1-4, wherein the stent (116) expands plastically.

6. The device of any of claims 1-2, wherein the second body (118) is an annular balloon.

7. The device of any of claims 1-6, further comprising a haemostatic coating (124) surrounding the second body (118).

8. The device any of claims 1-7, wherein the first and second bodies (110, 118) and the stent (116) extend along a majority length of the member (102).

9. The device of any of claims 1-8, wherein the elongated member (102) and the first body (110) are removable from the device.

10. The device of any of claims 1-9, wherein the elongated member (102) is axially rigid.

11. The device of any of claims 1-10, further comprising an emitter (140), configured to send navigational signals.

## Patentansprüche

1. Medizinische Vorrichtung (100), umfassend:
• ein langgestrecktes Element (102) mit einem proximalen Ende (103) und einem distalen Ende (101);
• eine ersten radial expandierbaren Körper (110), der das distale Ende (101) des Elementes (102) umgibt;
• einen den ersten Körper (110) umgebenden Stent (116), wobei die radiale Expansion des ersten Körpers (110) den Stent (116) radial expandiert; und
• einen den Stent (116) umgebenden zweiten radial expandierbaren Körper (118), wobei der erste und der zweite Körper (110, 118) unabhängig voneinander expandierbar sind.

2. Vorrichtung gemäß Anspruch 1, wobei das distale Ende (101) des Elementes (102) abgestumpft ist.

3. Vorrichtung gemäß Anspruch 1 oder 2, wobei der erste und der zweite Körper (110, 118) Ballons sind.

4. Vorrichtung gemäß Anspruch 1 oder 2, wobei der erste und der zweite Körper (110, 118) sich elastisch expandieren.

5. Vorrichtung gemäß irgendeinem der Ansprüche 1 bis 4, wobei der Stent (116) sich plastisch expandiert.

6. Vorrichtung gemäß irgendeinem der Ansprüche 1 bis 2, wobei der zweite Körper (118) ein ringförmiger Ballon ist.

7. Vorrichtung gemäß irgendeinem der Ansprüche 1 bis 6, ferner umfassend eine den zweiten Körper (118) umgebende haemostatische Beschichtung (124).

8. Vorrichtung gemäß irgendeinem der Ansprüche 1 bis 7, wobei der erste und zweite Körper (110, 118) und der Stent (116) sich entlang einem größeren Teil der Länge des Elementes (102) erstrecken.

9. Vorrichtung gemäß irgendeinem der Ansprüche 1 bis 8, wobei das langgestreckte Element (102) und der erste Körper (110) von der Vorrichtung entfernbar sind.

10. Vorrichtung gemäß irgendeinem der Ansprüche 1 bis 9, wobei das langgestreckte Element (102) in Axialrichtung steif ist.

11. Vorrichtung gemäß irgendeinem der Ansprüche 1 bis 10, ferner umfassend einen Emitter (140), der konfiguriert ist, Navigationssignale auszusenden.

## Revendications

1. Dispositif médical (100), comprenant:
• un élément longitudinal (102) avec une extrémité proximale (103) et une extrémité distale (101);
• un premier corps radialement expansible (110), qui entoure l'extrémité distale (101) de l'élément (102);
• un Stent (116) entourant le premier corps (110), la dilatation radiale du premier corps (110) radialement dilatant le Stent (116); et
• un deuxième corps radialement expansible (118), qui entoure le Stent (116), les corps premier et deuxième (110, 118) étant expansibles de façon indépendante l'un de l'autre.

2. Dispositif selon la revendication 1 dans lequel l'extrémité distale (101) de l'élément (102) est épointée.

3. Dispositif selon la revendication 1 ou 2, dans lequel les corps premier et deuxième (110, 118) sont des ballonnets.

4. Dispositif selon la revendication 1 ou 2, dans lequel les corps premier et deuxième (110, 118) se dilatent de façon élastique.

5. Dispositif selon l'une quelconque des revendications 1 à 4, dans lequel le Stent (116) se dilate de façon plastique.

6. Dispositif selon l'une quelconque des revendications 1 à 2, dans lequel le deuxième corps (118) est un ballonnet en forme d'anneau.

7. Dispositif selon l'une quelconque des revendications 1 à 6, comprenant en outre un revêtement hémostatique (124) entourant le deuxième corps (118).

8. Dispositif selon l'une quelconque des revendications 1 à 7, dans lequel les corps premier et deuxième (110, 118) et le Stent (116) s'étendent le long de la plus grande longueur de l'élément (102).

9. Dispositif selon l'une quelconque des revendications 1 à 8, dans lequel l'élément longitudinal (102) et le premier corps (110) sont amovibles du dispositif.

10. Dispositif selon l'une quelconque des revendications 1 à 9, dans lequel l'élément longitudinal (102) est rigide dans la direction axiale.

11. Dispositif selon l'une quelconque des revendications 1 à 10, comprenant en outre un émetteur (140) configuré à émettre des signaux de navigation.
